# EUROPÄISCHE PATENTSCHRIFT

(11) **EP 0 676 388 B1**
(45) Veröffentlichungstag und Bekanntmachung des Hinweises auf die Patenterteilung: **14.07.1999**
(21) Anmeldenummer: 95104285.2
(22) Anmeldetag: 23.03.1995
(51) Int. Cl.: C07C 51/34, C07C 57/32, C07C 17/26, C07C 22/04, C07C 43/303

(54) **Verfahren zur Herstellung von substituierten Phenylessigsäurederivaten und neue Zwischenprodukte**
Process for the preparation of phenylacetic acid derivatives and intermediate products
Procédé pour la préparation de dérivés substitués de l'acide phénylacétique et intermédiaires pour leur préparation

(30) Priorität: 05.04.1994 DE 4411667
(43) Veröffentlichungstag der Anmeldung: 11.10.1995
(60) Teilanmeldung: 98115966.8
(73) Patentinhaber: BAYER AG, 51368 Leverkusen (DE)
(72) Erfinder: Lantzsch, Reinhard, Dr., D-42115 Wuppertal (DE); Fuchs, Rainer, Dr., D-42113 Wuppertal (DE)

(56) Entgegenhaltungen:
- EP-A- 0 172 365
- EP-A- 0 173 019
- EP-A- 0 350 069
- US-A- 4 084 061
- CHEMICAL ABSTRACTS, vol. 53, no. 23, , Columbus, Ohio, US; abstract no. 21791a, DURGARYAN ET AL. 'condensation of 1,3-dichloro-2-butene with aromatic hydrocarbons in the presence of zinc chloride.' & NAUCH. TRUDY EREVAN. GOSUDARST. UNIV.; SER. CHIM. NAUK., Bd.53, Nr.3, Seiten 33 - 43

## Beschreibung

Die Erfindung betrifft neue Verfahren zur Herstellung von teilweise bekannten Phenylessigsäurederivaten und neue Zwischenprodukte zu ihrer Herstellung.

Es ist bekannt, daß man substituierte Phenylessigsäuren und ihre Derivate erhält, wenn man entsprechende substituierte Aromaten durch eine Chlormethylierung oder Brommethylierung in die Chlormethyl- oder Brommethylaromaten überführt, diese dann mit Cyaniden umsetzt und sie anschließend zur Säure verseift (vgl. z.B. J. Org. Chem. 58, Houben-Weyl, Methoden der Organischen Chemie, Georg Thieme Verlag, Stuttgart, Band 5/4 Seite 484, 1960, Houben-Weyl, Methoden der Organischen Chemie, Georg Thieme Verlag, Stuttgart, Band VIII, Seite 427, 1952). Dieses Verfahren weist jedoch den Nachteil auf, daß wegen der Möglichkeit der bei der Halogenmethylierung entstehenden kanzerogenen Bishalogenmethylether ein erhöhter Aufwand an Sicherheitsvorkehrungen bei der Reaktionsdurchführung erforderlich ist.

Aus JP-A-5000 4040 ist die oxidative Spaltung von Allylbenzol mit Natriumperiodat und Kaliumpermanganat beschrieben. In J. Org. Chem. 25, 1960, 108-111 wird die reduktive Spaltung von Ethylenderivaten mit Ozon zu Alkoholen beschrieben.

Weiter ist bekannt, daß man Phenylessigsäuren durch Carboxylierung von Benzylhalogeniden unter Phasentransferbedingungen erhält (Tetrahedron Lett., 24 (37), 4005-4008; J. Chem. Soc., Chem. Commun. (24), 1090-1091). Ein erheblicher Nachteil dieser Verfahren ist neben dem Einsatz von Eisen- und Cobaltcarbonylen, daß sie zum Teil unter Druck durchgeführt werden müssen und zu Reaktionsgemischen führen.

Gegenstand der vorliegenden Erfindung ist
1) ein Verfahren zur Herstellung von Phenylessigsäurederivaten der Formel (I) 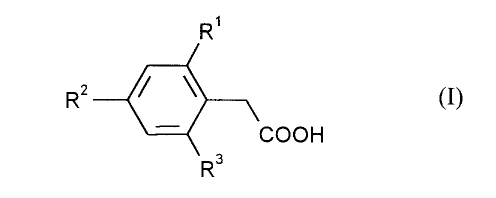 in welcher
   - R¹, R² und R³: jeweils unabhängig voneinander für Wasserstoff, Alkyl oder Alkoxy stehen,
   dadurch kennzeichnet, daß man substituierte Phenylpropene der Formel (II) 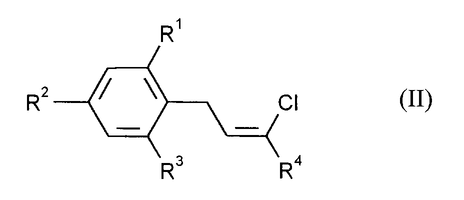 in welcher
   - R¹, R² und R³: die oben angegebene Bedeutung haben und
   - R⁴: für Wasserstoff oder Methyl steht, entweder
   a) in Gegenwart von inerten Lösungsmitteln einer Ozonolyse unterwirft, anschließend die so erhaltenen Aldehyde der Formel (III) 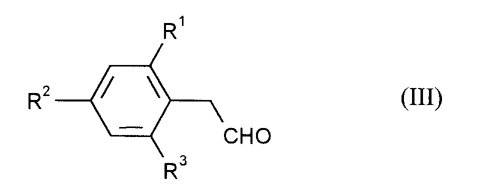 gegebenenfalls isoliert und diese dann in Gegenwart eines Verdünnungsmittels, in Gegenwart einer Säure und in Gegenwart eines Oxidationsmittels zu den Verbindungen der Formel (I) umsetzt,
      oder
   b) in Gegenwart von Alkoholen der Formel (IV)

      R⁵-OH (IV)

      in welcher
      R⁵ für Alkyl, insbesondere für C₁-C₄-Alkyl und ganz besonders für Methyl, Ethyl oder Butyl steht, einer Ozonolyse unterwirft und die so erhaltenen Phenylacetaldehydacetale der Formel (V) 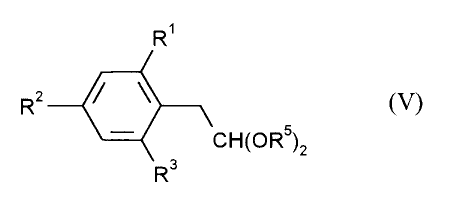 in welcher
      R¹, R², R³ und R⁵ die oben angegebene Bedeutung haben, gegebenenfalls isoliert und
      diese anschließend direkt oder nach Hydrolyse zu den Aldehyden der Formel (III), gegebenenfalls in Gegenwart eines Verdünnungsmittels in Gegenwart einer Säure und in Gegenwart eines Oxidationsmittels zu den Verbindungen der Formel (I) umsetzt.
2) Neue Mesitylacetaldehydacetale der Formel (Va) 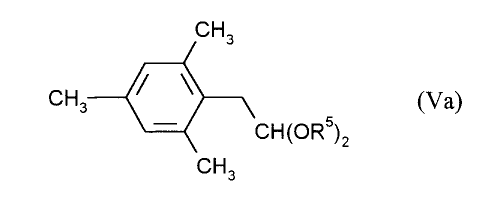 in welcher
   - R⁵: die oben angegebene Bedeutung hat.

Es ist als ausgesprochen überraschend anzusehen, daß nach dem erfindungsgemäßen Verfahren (1) die Phenylessigsäure-Derivate der Formel (I) nahezu quantitativ und in hoher Reinheit durch Ozonolyse und Oxidation erhalten werden, da bekannt ist, daß die sowohl bei Verfahren (1a), als auch bei Verfahren (1b) als Zwischenstufe entstehenden Arylacetaldehyde der Formel (III) sehr instabile Verbindungen sind und leicht mit sich selbst reagieren. Ferner können sich Arylessigsäuren leicht unter CO₂-Abspaltung zersetzen. Schließlich mußte der Fachmann eine Weiteroxidation zur substituierten Benzoesäure erwarten.

Ein weiterer Vorteil des erfindungsgemäßen Verfahrens ist, daß die Bildung des stark kanzerogenen Bishalogenmethylethers, der anfällt, wenn Phenylessigsäurederivate über die Zwischenstufe der Chlormethylierung von Aromaten hergestellt werden, vermieden wird. Das neue Verfahren kann somit unter deutlich verbesserten Sicherheits- und Umweltaspekten durchgeführt werden.

Nach dem erfindungsgemäßen Verfahren werden bevorzugt Verbindungen der Formel (I) hergestellt, in welcher
- R¹, R² und R³: jeweils unabhängig voneinander für Wasserstoff, C₁-C₄-Alkyl oder C₁-C₄-Alkoxy stehen.

Nach dem erfindungsgemäßen Verfahren werden besonders bevorzugt Verbindungen der Formel (I) hergestellt, in welcher
- R¹, R² und R³: jeweils unabhängig voneinander für Wasserstoff, Methyl, Ethyl, Methoxy oder Ethoxy stehen.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, als auch zwischen den jeweiligen Bereichen und Vorzugsbereichen, beliebig kombiniert werden.

Verwendet man beispielsweise für die erste Stufe 1-Chlor-3-(2,4,6-trimethylphenyl)-1-propen als Ausgangsstoff, Methylenchlorid als Lösungsmittel und Ozon und für die zweite Stufe Essigsäure und Wasserstoffperoxid, so läßt sich das erfindungsgemäße Verfahren (1a) durch das folgende Formelschema beschreiben:

### 1. Stufe

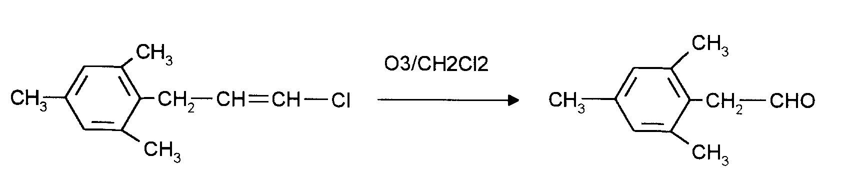

### 2. Stufe

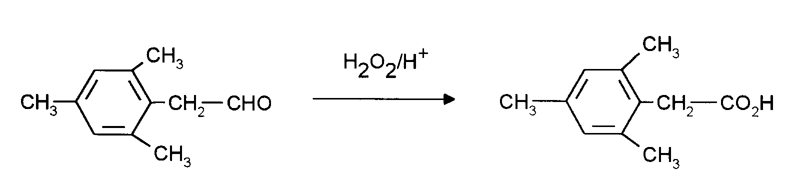

Verwendet man beispielsweise für die erste Stufe 1-Chlor-3-(2,4,6-trimethylphenyl)-1-propen und Methanol und Ozon als Ausgangsstoffe und für die zweite Stufe Essigsäure und Wasserstoffperoxid, so läßt sich das erfindungsgemäße Verfahren (1b) durch das folgende Formelschema beschreiben:

### 1. Stufe

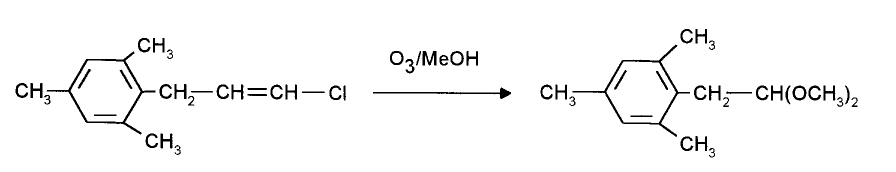

### 2. Stufe

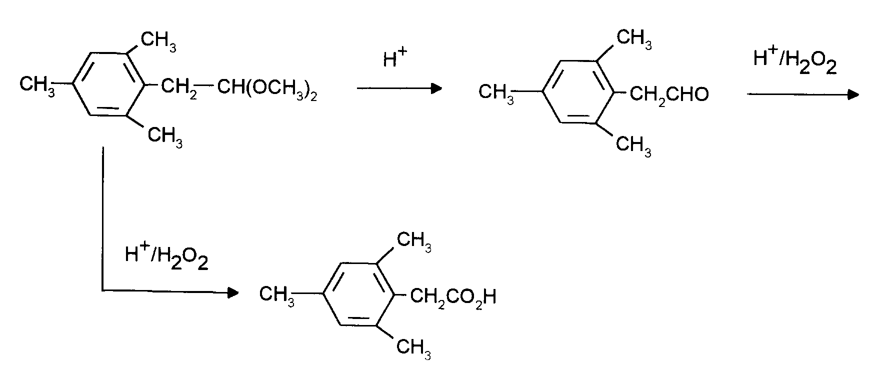

Die bei dem oben unter (1) angegebenen erfindungsgemäßen Verfahren zur Herstellung der Verbindungen der Formel (I) als Ausgangsstoffe zu verwendenden substituierten Phenylpropene sind durch die Formel (II) allgemein definiert. In der Formel (II) stehen R¹, R² und R³ vorzugsweise bzw. insbesondere für diejenige Bedeutung, die bereits oben im Zusammenhang mit der Beschreibung der Verbindungen der Formel (I) als bevorzugt bzw. insbesondere bevorzugt für R¹, R² und R³ angegeben wurde. R⁴ steht für Wasserstoff oder Methyl.

Die Verbindungen der Formel (II) sind bekannt oder können nach bekannten Verfahren hergestellt werden (vgl. Comptes Rendus 213, 619-620 (1941)). Die bekannten und die noch nicht bekannten Verbindungen der Formel (II) können beispielsweise nach dem unter (2) beschriebenen erfindungsgemäßen Verfahren erhalten werden.

Die bei dem oben unter (1b) angegebenen Verfahren außerdem als Ausgangsstoffe zu verwendenden Alkohole sind durch die Formel (IV) allgemein definiert. In dieser Formel (IV) hat R⁵ bevorzugt bzw. besonders bevorzugt diejenige Bedeutung, die bereits oben im Zusammenhang mit der Beschreibung der Verbindungen der Formel (IV) als bevorzugt bzw. besonders bevorzugt für R⁵ angegeben wurde.

Die Alkohole der Formel (IV) sind allgemein bekannte Verbindungen der organischen Chemie.

Die bei dem oben unter (1b) angegebenen Verfahren als Zwischenprodukte erhältlichen Phenylacetaldehydacetale sind durch die Formel (V) allgemein definiert. In dieser Formel (V) stehen R¹, R², R³ und R⁵ vorzugsweise bzw. insbesondere für diejenige Bedeutung, die bereits oben im Zusammenhang mit der Beschreibung der Verbindungen der Formeln (I) und (IV) als bevorzugt bzw. insbesondere bevorzugt für R¹, R², R³ und R⁵ angegeben wurde.

Einige der Phenylacetaldehydacetale der Formel (V) sind bereits prinzipiell aus der Literatur bekannt (vgl. z.B. EP 403 841, FR 2 577 920). Die bekannten und die noch nicht bekannten Phenylacetaldehydacetale der Formel (V) können nach dem unter (1b) beschriebenen erfindungsgemäßen Verfahren erhalten werden.

Die bei dem oben unter (1) angegebenen Verfahren weiterhin als Zwischenprodukte erhältlichen Aldehyde der Formel (III) sind allgemein bekannte Verbindungen der organischen Chemie.

Das unter (1a) beschriebene erfindungsgemäße Verfahren wird vorzugsweise in Gegenwart eines Verdünnungsmittels durchgeführt.

Als Verdünnungsmittel zur Durchführung des unter (1a) beschriebenen Verfahrens kommen alle unter den Reaktionsbedingungen inerten, üblichen organischen Lösungsmittel in Betracht. Hierzu gehören beispielsweise insbesondere inerte Solventien: beispielsweise Chlorkohlenwasserstoffe wie Methylenchlorid, Ketone, wie Aceton, Ester wie Essigsäuremethyl- oder -ethylester und Kohlenwasserstoffe wie Pentan, Hexan oder Cyclohexan.

Die Reaktionstemperaturen können bei der Durchführung der unter (1a) und (1b) beschriebenen erfindungsgemäßen Verfahren in einem größeren Bereich variiert werden. Im allgemeinen arbeitet man bei Temperaturen zwischen -70°C und +20°C, vorzugsweise zwischen -30°C und 0°C.

Das unter (1b) beschriebene erfindungsgemäße Verfahren wird vorzugsweise in Gegenwart von Alkoholen durchgeführt. Als Alkohole zur Durchführung des unter (1b) beschriebenen Verfahrens kommen primäre Alkohole, insbesondere Methanol, Ethanol oder n-Butanol in Betracht.

Das unter (1a) beschriebene erfindungsgemäße Verfahren wird so durchgeführt, daß das entsprechende substituierte Phenylpropen der Formel (II) in einem der oben angegebenen Lösungsmittel vorgelegt wird und danach bei der angegebenen Temperatur so lange Ozon eingeleitet wird, bis die Reaktion beendet ist. Das überschüssige Ozon wird mit einem Inertgas, wie beispielsweise Stickstoff oder Argon ausgeblasen oder mit einem Reduktionsmittel, wie beispielsweise Dimethylsulfid oder Tetrahydrothiophen zerstört, wobei gleichzeitig das gebildete Ozonid gespalten wird. Statt Sulfiden können auch andere Reduktionsmittel verwendet werden, wie beispielsweise Thioharnstoff, Natriumhydrogensulfitlösung, Zinkstaub in Essigsäure. Die Spaltung gelingt auch durch Wasserstoff mittels katalytischer Hydrierung nach allgemein bekannten Methoden. Ferner ist die Verwendung von Aminen, wie beispielsweise Triethylamin, zur Spaltung des Primärozonides möglich.

Das unter (1b) beschriebene erfindungsgemäße Verfahren wird analog dem oben unter (1a) beschriebenen Verfahren, jedoch in Gegenwart von Alkoholen als Lösungsmittel durchgeführt. Man erhält dann die Phenylacetaldehydacetale der Formel (V), welche nach üblichen Methoden, beispielsweise durch saure Hydrolyse in die Aldehyde der Formel (III) übergeführt werden können (siehe Linke in Houben-Weyl, Band E3, Aldehyde, Seite 362 - 367, 1983).

Es kann gegebenenfalls auch vorteilhaft sein, die nach dem erfindungsgemäßen Verfahren (1a) und (1b) entstandenen Aldehyde der Formel (III) und die Phenylacetaldehydacetale der Formel (V) nicht zu isolieren, sondern nach dem Abdestillieren des Lösungsmittels in einer Eintopfreaktion mit Wasserstoffperoxid-Lösung, vorzugsweise unter Zusatz von Säuren, wie beispielsweise Essigsäure oder Propionsäure, direkt zu den Phenylessigsäuren der Formel (I) weiter umzusetzen. Die Phenylessigsäuren werden durch Extraktion der wäßrigen Phase oder durch Abfiltrieren erhalten.

Die unter (2) angegebenen Mesitylacetaldehyde sind durch die Formel (Va) allgemein definiert.
- R⁵: steht bevorzugt für C₁-C₄-Alkyl, besonders bevorzugt für Methyl, Ethyl und n-Butyl.

Die Mesitylacetaldehydacetale der Formel (Va) sind neu und Gegenstand der Erfindung. Sie können nach dem erfindungsgemäßen Verfahren (1b) erhalten werden.

Die nach dem erfindungsgemäßen Verfahren (1) herzustellenden Phenylessigsäurederivate der Formel (I) können als Ausgangsstoffe zur Herstellung von Schädlingsbekämpfungsmitteln verwendet werden (vgl. z.B. EP-A 528 156).

Die Erfindung soll durch die folgenden Beispiele verdeutlicht werden:

### Beispiele

### Beispiel 1

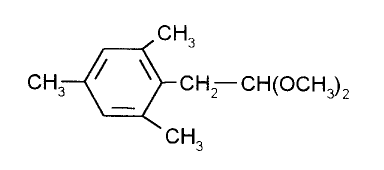

1,95g (0,01 Mol) 1-Chlor-3-(2,4,6-trimethylphenyl)-prop-1-en werden in 60ml Methanol gelöst und auf -30°C abgekühlt. Danach wird mittels eines Ozongenerators erzeugtes Ozon innerhalb von 40 Minuten durch das Reaktionsgemisch geleitet, wobei sich die klare, farblose Lösung leicht grau färbt. Der Fortgang der Oxidation wird durch Dünnschicht- oder Gaschromatographie verfolgt. Nach vollständigem Umsatz wird das gebildete Ozonid mit Dimethylsulfid gespalten und das Methanol im Vakuum abdestilliert. Man erhält praktisch quantitativ das Dimethylacetal des 2,4,6-Trimethylphenylacetaldehyds als Rohprodukt, welches durch Destillation im Kugelrohr (2mbar, Manteltemperatur 80 - 100°C) weiter gereinigt werden kann, Abtrennung des aus Dimethylsulfid gebildeten Dimethylsulfoxids.

Das Produkt wurde durch das Massenspektrum charakterisiert: ^{m}/ₑ = 208 (Molpeak), 177, 147, 133, 75 (basepeak), 47.

### Beispiel 2

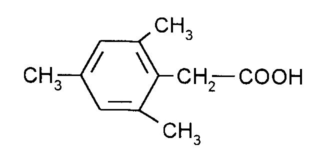

Das nach Beispiel 1 erhaltene Rohprodukt des Dimethylacetals des 2,4,6-Trimethylphenylacetaldehyds wird mit 3,5ml Wasser, 4ml Essigsäure und 2ml 30%igem Wasserstoffperoxid versetzt und bei 25 - 35°C 12 Stunden gerührt. Nach Eindampfen bis zur Trockne wird in Wasser/Ethanol aufgenommen. Dann filtriert man ab und trocknet.

Man erhält 1,45g (81% der Theorie) 2,4,6-Trimethylphenylessigsäure vom Schmelzpunkt 165°C.

### Beispiel 3

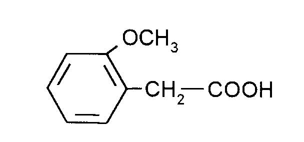

2g (0,01 Mol) 2-Chlor-3-(2'-methoxy-phenyl)-2-buten (bekannt aus C. A. 73 (13), 66 192 c) werden in 60ml Methylenchlorid gelöst und auf -50°C abgekühlt. Danach wird mittels eines Ozongenerators erzeugten Ozon innerhalb von 45 Minuten durch das Reaktionsgemisch geleitet. Nach beendeter Reaktion wird überschüssiges Ozon mit Stickstoff ausgetrieben und das gebildete Ozonid mit 2 g (0,02 Mol) Triethylamin versetzt. Man läßt auf Raumtemperatur kommen, wäscht die organische Phase mit Wasser und dampft ein. Der Rückstand wird mit 5 ml Eisessig und bei 60°C mit 3ml 30%iger Wasserstoffperoxidlösung versetzt und 2 Stunden gerührt. Nach dem Abkühlen wird mit Wasser verdünnt und dreimal mit Methylenchlorid extrahiert.

Nach dem Trocknen und Abdestillieren erhält man 1,5 g (90% der Theorie) 2-Methoxyphenylessigsäure vom Schmelzpunkt 123°C.

## Patentansprüche

1. Verfahren zur Herstellung von Phenylessigsäurederivaten der Formel (I) 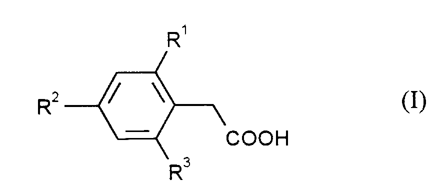 in welcher
R¹, R² und R³ jeweils unabhängig voneinander für Wasserstoff, Alkyl oder Alkoxy stehen,
dadurch kennzeichnet, daß man substituierte Phenylpropene der Formel (II) 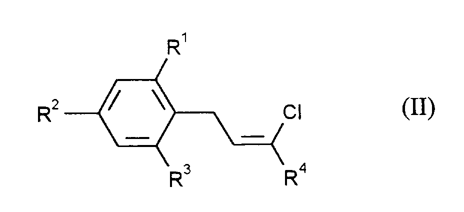 in welcher
R¹, R² und R³ die oben angegebene Bedeutung haben und
R⁴ für Wasserstoff oder Methyl steht, entweder
a) in Gegenwart von inerten Lösungsmitteln einer Ozonolyse unterwirft, anschließend die so erhaltenen Aldehyde der Formel (III) 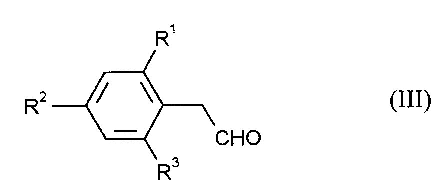 gegebenenfalls isoliert und diese dann in Gegenwart eines Verdünnungsmittels, in Gegenwart einer Säure und in Gegenwart eines Oxidationsmittels zu den Verbindungen der Formel (I) umsetzt,
oder
b) in Gegenwart von Alkoholen der Formel (IV)
R⁵-OH (IV)
in welcher
R⁵ für Alkyl steht,
einer Ozonolyse unterwirft und die so erhaltenen Phenylacetaldehydacetale der Formel (V) 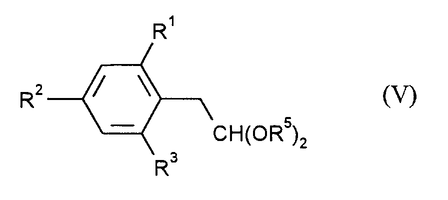 in welcher
R¹, R², R³ und R⁵ die oben angegebene Bedeutung haben,
gegebenenfalls isoliert und
diese anschließend direkt oder nach Hydrolyse zu den Aldehyden der Formel (III), gegebenenfalls in Gegenwart eines Verdünnungsmittels in Gegenwart einer Säure und in Gegenwart eines Oxidationsmittels zu den Verbindungen der Formel (I) umsetzt.

2. Verbindungen der Formel (Va) 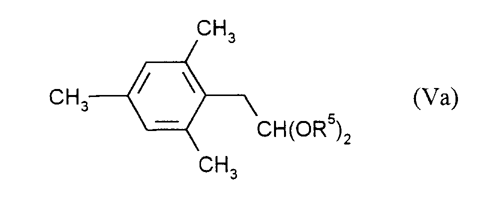 in welcher
R⁵ für Alkyl steht.

## Claims

1. Process for the preparation of phenylacetic acid derivatives of the formula (I) 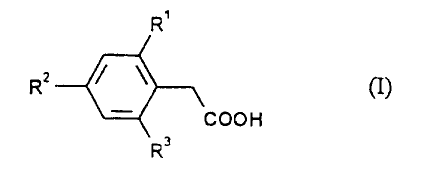 in which
R¹, R² and R³ independently of each other each represent hydrogen, alkyl or alkoxy,
characterized in that substituted phenylpropenes of the formula (II) 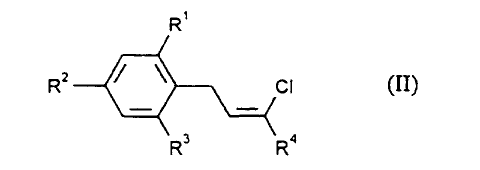 in which
R¹, R² and R³ have the meaning given above and
R⁴ represents hydrogen or methyl, are subjected either
a) to an ozonolysis in the presence of inert solvents, then if appropriate the aldehydes of the formula (III) thus obtained are isolated and then these are reacted in the presence of a diluent, in the presence of an acid and in the presence of an oxidizing agent to give the compounds of the formula (I),
or
b) to an ozonolysis in the presence of alcohols of the formula (IV)
R⁵-OH (IV)
in which
R⁵ represents alkyl,
and if appropriate the phenylacetaldehyde acetals of the formula (V) thus obtained 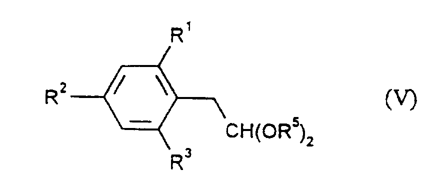 in which
R¹, R², R³ and R⁵ have the meaning given above
are isolated and
then these are reacted to give the compounds of the formula (I) directly or after hydrolysis to give the aldehydes of the formula (III), in the presence or absence of a diluent in the presence of an acid and in the presence of an oxidizing agent.

2. Compound of the formula (Va) 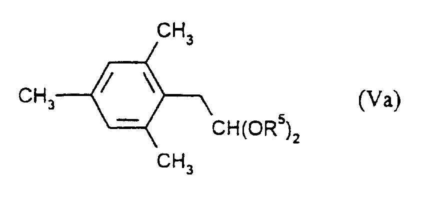 in which
R⁵ represents alkyl.

## Revendications

1. Procédé de production de dérivés d'acide phénylacétique de formule (I) 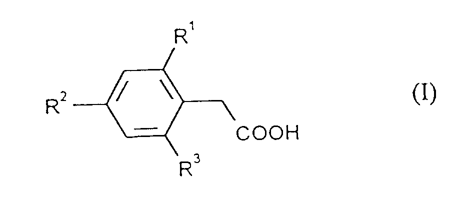 dans laquelle
R¹, R² et R³ représentent chacun, indépendamment les uns des autres, de l'hydrogène, un groupe alkyle ou un groupe alkoxy,
caractérisé en ce que des phénylpropènes substitués de formule (II) 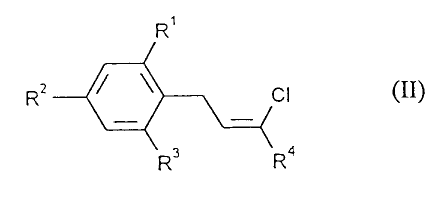 dans laquelle
R¹, R² et R³ ont la définition indiquée ci-dessus et
R⁴ est l'hydrogène ou un groupe méthyle,
a) sont soumis à une ozonolyse en présence de solvants inertes, puis les aldéhydes ainsi obtenus de formule (III) 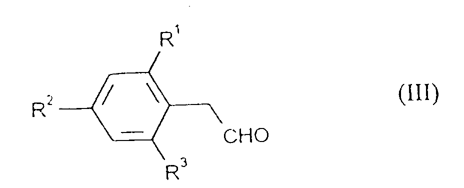 sont éventuellement isolés puis sont convertis en présence d'un diluant, en présence d'un acide et en présence d'un agent oxydant, en les composés de formule (I),
ou bien
b) sont soumis à une ozonolyse en présence d'alcools de formule (IV)
R⁵-OH (IV)
dans laquelle
R⁵ est un groupe alkyle,
et les phénylacétaldéhyde-acétals ainsi obtenus de formule (V) 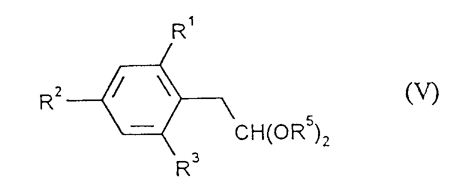 dans laquelle
R¹, R², R³ et R⁵ ont la définition indiquée ci-dessus,
sont éventuellement isolés et
ces composés sont convertis en les composés de formule (I), directement ou après hydrolyse en aldéhydes de formule (III), le cas échéant en présence de diluants, en présence d'un acide et en présence d'un agent oxydant.

2. Composés de formule (Va) 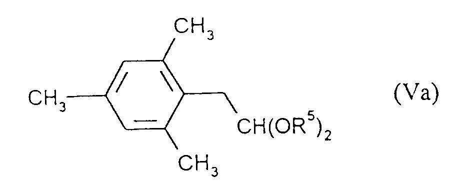 dans laquelle
R⁵ est un groupe alkyle.
